# EUROPEAN PATENT SPECIFICATION

(11) **EP 4 237 826 B1**
(45) Date of publication and mention of the grant of the patent: **24.12.2025**
(21) Application number: 21798310.5
(22) Date of filing: 18.10.2021
(51) Int. Cl.: G01N 21/47, G01N 21/85, G01N 21/15, G01N 33/18

(54) **SELF CLEANING OPTICAL PROBE**
SELBSTREINIGENDE OPTISCHE SONDE
SONDE OPTIQUE AUTONETTOYANTE

(30) Priority: 30.10.2020 GB 202017232
(43) Date of publication of application: 06.09.2023
(73) Proprietor: Inov8 Systems Limited, Belfast, Antrim BT3 9JQ (GB)
(72) Inventor: THABETH, Khalid, Belfast, Antrim BT3 9JQ (GB); ACHESON, Raymond, Belfast, Antrim BT3 9JQ (GB); THABETH, Zaid, Belfast, Antrim BT3 9JQ (GB)
(74) Representative: FRKelly
(86) International application number: PCT/EP2021/078775
(87) International publication number: WO 2022/089976

(56) References cited:
- WO-A1-2007/096179
- DE-A1- 4 333 560
- JP-A- H01 320 449

## Description

### FIELD OF THE INVENTION

This invention relates to a self cleaning optical probe and in particular to a self cleaning optical probe for water analysers.

### BACKGROUND OF THE INVENTION

There are many applications that require the analysis of water to identify substances present. For example, in municipal water there is a requirement to ensure that the amounts of certain substances within the water are below specified levels. When discharging waste water from shipping and industry in general there is a requirement to ensure that amounts of specified substances in the waste water do not exceed predetermined limits. Water analysers or probes are used for this purpose, either in side stream passages or as insertion probes.

Many substances have a natural fluorescence. Therefore, water analysers often measure the quantity of these substances present in water by the detection of fluorescence. Devices that detect and/or measure fluorescence are commonly referred to as fluorometers. A fluorometer usually includes a light source for causing fluorescence in a target substance and a detector for measuring the resultant fluorescence.

A typical in-line or side stream water analyser has a measurement window forming a portion of a wall of a measurement region through which the excitation light source is transmitted into the measurement region and through which the resultant fluorescent and/or reflected light is received to be analysed in order to determine the quantity and/or identify of target substances and/or other contaminants present.

In insertion probes, a measurement window is provided at a distal end of the probe.

One problem with such devices is the fouling of the measurement window by substances within the measurement region. This problem may be addressed by using an ultrasonic transducer to transit ultrasonic energy through the measurement window, whereby the measurement window can be cleaned by ultrasonic cavitation created in the fluid within the measurement region by the ultrasonic energy transmitted through the measurement window from the ultrasonic transducer.

A known optical probe of a water analyser comprises an elongate hollow probe shaft having a measurement window at a distal end thereof. An ultrasonic transducer is mounted on an opposite end of the probe shaft, for example having transducer discs located between a back mass and the probe shaft. A bolt typically passes through the rear of the back mass and through the ceramic transducer discs into the rear end of the probe shaft to secure the ceramic discs and back mass to the probe shaft.

The location of the measurement window directly within the ultrasonic transducer, or at least in the path of the ultrasonic energy generated by the ultrasonic transducer, leads to mechanical stressing of the measurement window and erosion and etching of the window due to the direct exposure of the window to the powerful ultrasonic energy and due to the intense cavitation generated in the fluid at the surface of the measurement window. Etching of a measurement window due to cavitation is a well known limitation or side effect of using ultrasonic energy to clean such measurement windows. This etching may not only affect the light traveling through the window, which in turn directly affects the instrument measurement capabilities, but may also compromise the mechanical integrity of the window, leading to fracture and potentially water ingress.

Furthermore, in many applications, light from the light source is both transmitted through the measurement window into the measurement region and returns from the measurement region through the same measurement window. The use of a common measurement window for both the transmit and receive paths can lead to internal reflections, reducing sensitivity of the instrument and increasing noise. DE4333560, JPH01320449 and WO2007/096179A1 show known optical probes.

### SUMMARY OF THE INVENTION

According to the present invention there is provided an optical probe as claimed in claim 1.

By locating the first and second light guides entirely outside of the ultrasonic energy path the damaging etching effect caused by direct cavitation on the outer faces of the lights guides is avoided. The front faces of the lights guides may define respective measurement windows through which light passes into and out of the measurement region.

In a preferred embodiment said at least one first light guide and at least one second light guide may be mounted within said front plate on respective axes converging with a common point within said measurement region.

Preferably said at least one first light guide and said at least one second light guide each comprise an elongate rod mounted within a respective receiving channel extending through said front plate. Each of said at least one first light guide and said at least one second light guide may be configured to facilitate the correct location and/or orientation of the respective light guide within its receiving channel in the front plate, for example via cooperating formations on the outer surfaces of the light guide and inner faces of its respective receiving channel.

The ultrasonic transducer may be coupled to said front plate via an elongate body, at least a distal end of said elongate body having a diameter less than the diameter of said front plate such that said elongate body cooperates with a central region of said front plate to transmit ultrasonic vibrations thereto. Said at least one first light guide and said at least one second light guide may be located within a peripheral region of said front plate outside of said central region thereof. The ultrasonic transducer may be mounted against a first end of said elongate body, a second end of said elongate body, opposite said first end, being mounted against said front plate. The ultrasonic transducer may be secured to said first end of said elongate body by means of a fastener passing therethrough. The second end of said elongate body may be secured to said front plate by means of a fastener passing therethrough. The ultrasonic transducer may comprise a stack of transducer discs coupled to a back mass.

Said at least one first light guide and/or said at least one second light guide may be adapted to modify light passing therethrough. Said at least one first light guide and/or said at least one second light guide may comprise or incorporate at least one lens. In one embodiment a distal end of each of said at least one first light guide and said at least one second light guide may be adapted to modify light passing therethrough. For example, the distal end of each said at least one first light guide and said at least one second light guide may comprise a face extending at an acute angle to the axis of the respective light guide and substantially parallel to said front face of the front plate and wherein said at least one first light guide and said at least one second light guide are mounted within said front plate on respective axes arranged to converge on a common point within the measurement region.

In one embodiment each of said at least one first light guide and said at least one second light guide may comprise coaxially arranged first and second parts, the first part being mounted in the front plate such that a distal end thereof communicates with the measurement region, the second part being mounted adjacent to and coaxially aligned with the respective first part, a gap being provided between adjacent ends of the first and second parts of each of said at least one first light guide and said at least one second light guide to thereby isolate the second part of each of said at least one first light guide and said at least one second light guide from ultrasonic energy. The gap provided between adjacent ends of the first and second parts of each of said at least one first light guide and said at least one second light guide may be filled with an optically transmissive medium, such as optical coupling fluid, preferably having a refractive index identical or similar to the first and second parts of the respective light guide. The second part of each of said at least one first light guide and at least one second light guide may be mounted with a housing part coupled to the front plate. The housing part is preferably coupled to the front plate so as to be substantially isolated from ultrasonic energy from the ultrasonic transducer. In one embodiment, where the ultrasonic transducer includes an elongate body coupled to the front pate, the housing part may be coupled to a peripheral region of the front plate, said elongate body extending through an opening in said housing part such that the elongate body is not in direct contact with the housing part.

Preferably each of said at least one first light guide and each of said at least one second light guide comprises a sapphire rod or other suitable optical rod.

### BRIEF DESCRIPTION OF THE DRAWINGS

A self cleaning optical probe in accordance with an embodiment of the present invention will now be illustrated, by way of example, with reference to the accompanying drawings, in which:
Figure 1 is a longitudinal sectional view through an optical probe in accordance with a first embodiment of the present invention; and
Figure 2 is a longitudinal sectional view through a front part of the optical probe of Figure 1.

### DETAILED DESCRIPTION OF THE DRAWINGS

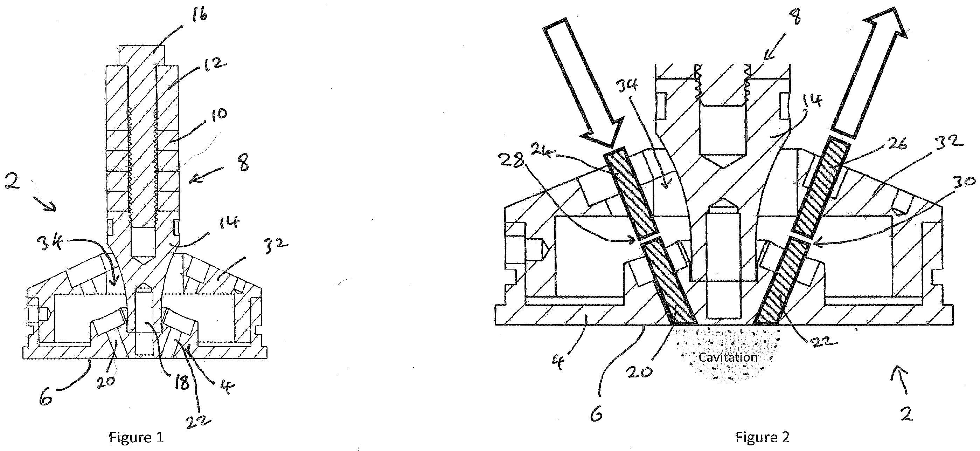

As illustrated in Figure 1, an optical probe 2 in accordance with an embodiment of the present invention comprises a front plate 4 defining a distal end of an insertion probe or a wall portion of a measurement region of a side stream water analyser, a front or outer face 6 of said front plate 4 bounding the measurement region.

An ultrasonic transducer 8 is provided for transmitting ultrasonic energy to the front plate in order to transmit ultrasonic energy through a central region of the front plate 4 and generate cavitation within a defined zone of cavitation in the measurement region adjacent the central region of the front plate 4.

The ultrasonic transducer comprises a stack of ceramic transducer discs 10 located between a back mass 12 and an elongate transducer body 14. A bolt 16 passes through the rear of the back mass 12 and through the ceramic transducer discs 10 into the rear end of the transducer body 14 to secure the ceramic discs 10 and back mass 12 to the transducer body 14. The transducer body 14 of the ultrasonic transducer 8 is mechanically coupled to the front plate 4, for example by means of a bolt or other threaded fastener 18, such that the ultrasonic transducer transits ultrasonic energy along an energy path extending through the central region of the front plate 4.

Light guides 20,22, preferably in the form of sapphire rods, are mounted in respective receiving channels within the front plate 4 to transmit and received light into and out of the measurement region. The light guides 20,22 are mounted in the front plate 4 within a peripheral region of said front plate outside of said central region thereof, outside of said energy path, such that the light guides 20,22 are not directly exposed to ultrasonic energy from the transducer 8. However, the distal end of each of the light guides 20,22 is positioned in the front plate 4 to be exposed at the outer face 6 of the front plate 4 within the measurement region and within the zone of cavitation such that said cavitation serves to clean an outer surface of said distal end of each light guide 20,22. More specifically, the light guides 20,22 extend through the front plate at respective locations spaced from the energy path of the ultrasonic transducer 8 such that the light guides are not exposed to direct ultrasonic energy from said ultrasonic transducer while being exposed to said zone of cavitation generated by the ultrasonic transducer.

The location of the light guides 20,22 to the sides of the central region of the front plate 4 advantageously increases the useful life of the light guides 20,22, the light guides 20,22 being substantially isolated from the ultrasonic energy from the ultrasonic transducer 8 and thereby incurring minimal erosion compared to the measurement window of prior art optical probes. Furthermore, the use of separate light guides 20,22 for the transmission and reception of light into and out of the measurement region eliminates the problem of internal reflections commonly seen when using a single measurement window, greatly improving the signal to noise ratio, improving the sensitivity of the instrument.

While the drawings show a single pair of light guides 20,22 for the transmission and reception of light into and out of the measurement region, it is envisaged that multiple pairs of light guides may be mounted within the front plate, radially spaced around the transducer body 14, each pair of light guides preferably lying in a common plane coincident with the axis of the transducer body.

The sapphire rods defining the light guides 20,22 may be adapted modify the light passing into and out of the light guides 20,22, as required, such that the light can be focussed or redirected towards specific locations within the measurement region, for example towards a central portion of the measurement region. In the embodiment shown the light guides are mounted within the front plate 4 on respective axes converging with a common point within the measurement window, with a distal end face of each light guide being arranged parallel to the front face 6 of the front plate 4.

In a preferred embodiment, as illustrated in the drawings, each of the light guides comprises coaxially arranged first and second parts, each first part 20,22 comprising a respective sapphire rod mounted in the front plate 4 such that a distal end thereof communicates with the measurement region, each second part 24,26, preferably comprising a separate sapphire rod, being mounted adjacent to and coaxially arranged with the respective first part 20,22, such that a gap 28,30 is provided between adjacent ends of the first and second parts of each light guide to thereby isolate the second part of each light guide from any ultrasonic energy which might be passed thereto from the first part. The second part of each light guide may comprise a distal end of an optic fibre.

The gap 28,30 provided between adjacent ends of the first and second parts of each light guide may be filled with an optically transmissive medium, such as an optical coupling fluid, preferably having a refractive index identical or similar to that of the light guides.

In the embodiment shown in the drawings, the second part 24,26 of each light guide is mounted with a housing part 32 located behind and coupled to the front plate 4. The housing part 32 is preferably coupled to the front plate 4 so as to be substantially isolated from ultrasonic energy from the ultrasonic transducer 8, for example being coupled to a peripheral region of the front plate 4, remote from the zone of cavitation. The transducer body 14 preferably extends through a central opening 34 in said housing part 32 such that the transducer body 14 is not in direct contact with the housing part 32.

Each light guide or part thereof, or at least the first part 20,22 of each light guide, may be mounted within a respective receiving channel extending through said front plate 4 and/or the housing part 32, the outer surface thereof being configured to facilitate the correct location and/or orientation of the respective light guide or part thereof within its respective receiving channel. For example, the outer surface of each light guide or part thereof may incorporate projections or recesses cooperating with corresponding surface features of the respective receiving channel and/or may include stepped portions having of differing radius or other surface features arranged to locate each light guide or respective part thereon within its respective receiving channel and/or to ensure correct orientation thereof within the respective receiving channel.

An optical probe construction in accordance with the present invention facilitates the insertion of numerous devices e.g. optical sensors, cameras, light sources etc. effectively creating a generic self cleaning carrier probe that will facilitate the insertion of various devices into fluid environments, negating the need for additional routine cleaning.

The invention is not limited to the embodiments described herein but can be amended or modified without departing from the scope of the present invention as defined in the appended claims.

## Claims

1. An optical probe (2) comprising a front plate (4) having a front face bounding a measurement region, at least one first light guide (20) being mounted within said front plate (4) for transmitting light directly into said measurement region, at least one second light guide (22) being mounted within said front plate (4) for receiving light directly transmitted from said measurement region, wherein an ultrasonic transducer (8) is coupled to said front plate (4) to transmit ultrasonic vibrations from said ultrasonic transducer to said front plate (4) along an energy path to generate cavitation in said measurement region, wherein said at least one first light guide (20) and at least one second light guide (22) extend through the front plate (4) at respective locations spaced from said energy path such that said first and second light guides (20,22) are not exposed to direct ultrasonic energy from said ultrasonic transducer (8) while being exposed to a zone of cavitation generated by the ultrasonic transducer (8).

2. An optical probe as claimed in claim 1, wherein said at least one first light guide (20) and at least one second light guide (22) are mounted within said front plate (4) on respective axes converging with a common point within said measurement region.

3. An optical probe as claimed in claim 1 or claim 2, wherein said at least one first light guide (20) and said at least one second light guide (22) each comprise an elongate rod mounted within a respective receiving channel extending through said front plate (4).

4. An optical probe as claimed in claim 3, wherein each of said at least one first light guide (20) and at least one second light guide (22) is configured to facilitate the correct location and/or orientation of the respective light guide within its receiving channel in the front plate (4).

5. An optical probe as claimed in any preceding claim, wherein said ultrasonic transducer (8) is coupled to said front plate (4) via an elongate body (14), at least a distal end of said elongate body (14) having a diameter less than the diameter of said front plate (4) such that said elongate body (14) cooperates with a central region of said front plate (4) to transmit ultrasonic vibrations thereto.

6. An optical probe as claimed in claim 5, wherein said at least one first light guide (20) and said at least one second light guide (22) are located within a peripheral region of said front plate (4) outside of said central region thereof.

7. An optical probe as claimed in claim 5 or claim 6, wherein said ultrasonic transducer (8) is mounted against a first end of said elongate body (14), a second end of said elongate body (14), opposite said first end, being mounted against said front plate (4).

8. An optical probe as claimed in claim 7, wherein said ultrasonic transducer (8) is secured to said first end of said elongate body (14) by means of a fastener (16) passing therethrough.

9. An optical probe as claimed in claim 7 or claim 8, wherein said second end of said elongate body (14) is secured to said front plate (4) by means of a fastener (18) passing therethrough.

10. An optical probe as claimed in any of claims 7 to 9, wherein said ultrasonic transducer (8) comprises a stack of transducer discs (10) coupled to a back mass (12).

11. An optical probe as claimed in any preceding claim, wherein said at least one first light guide (20) and/or said at least one second light guide (22) is adapted to modify light passing through it.

12. An optical probe as claimed in claim 11, wherein said at least one first light guide (20) and/or said at least one second light guide (22) comprises or incorporates at least one lens.

13. An optical probe as claimed in claim 12, wherein a distal end of each of said at least one first light guide (20) and said at least one second light guide (22) is adapted to modify light passing therethrough.

14. An optical probe as claimed in claim 13, wherein said distal end of each said at least one first light guide (20) and said at least one second light guide (22) comprises a face extending at an acute angle to the axis of the respective light guide and substantially parallel to said front face (6) of the front plate (4) and wherein said at least one first light guide (20) and said at least one second light guide (22) are mounted within said front plate (20) on respective axes arranged to converge on a common point within the measurement region.

15. An optical probe as claimed in any preceding claim, wherein each of said at least one first light guide (20) and said at least one second light guide (22) comprises coaxially arranged first and second parts, the first part being (20,22) mounted in the front plate (4) such that a distal end thereof communicates with the measurement region, the second part (24,26) being mounted adjacent to and coaxially aligned with the respective first part (20,22), a gap (28,30) being provided between adjacent ends of the first and second parts of each of said at least one first light guide and said at least one second light guide to thereby isolate the second part (24,26) of each of said at least one first light guide (20) and said at least one second light guide (22) from ultrasonic energy.

## Patentansprüche

1. Optische Sonde (2), umfassend eine Frontplatte (4), die eine Frontfläche aufweist, die einen Messbereich begrenzt, wobei mindestens ein erster Lichtleiter (20) innerhalb der Frontplatte (4) zum Übertragen von Licht direkt in den Messbereich angebracht ist, wobei mindestens ein zweiter Lichtleiter (22) innerhalb der Frontplatte (4) zum Empfangen von Licht, das direkt von dem Messbereich übertragen wird, angebracht ist, wobei ein Ultraschallwandler (8) an die Frontplatte (4) gekoppelt ist, um Ultraschallschwingungen entlang eines Energiepfads von dem Ultraschallwandler auf die Frontplatte (4) zu übertragen, um eine Kavitation in dem Messbereich zu erzeugen, wobei sich der mindestens eine erste Lichtleiter (20) und der mindestens eine zweite Lichtleiter (22) an jeweiligen von dem Energiepfad beabstandeten Stellen durch die Frontplatte (4) erstrecken, sodass der erste und der zweite Lichtleiter (20, 22) keiner direkten Ultraschallenergie von dem Ultraschallwandler (8) ausgesetzt sind, während sie einer durch den Ultraschallwandler (8) erzeugten Kavitationszone ausgesetzt sind.

2. Optische Sonde nach Anspruch 1, wobei der mindestens eine erste Lichtleiter (20) und der mindestens eine zweite Lichtleiter (22) innerhalb der Frontplatte (4) an jeweiligen Achsen angebracht sind, die in einem gemeinsamen Punkt innerhalb des Messbereichs konvergieren.

3. Optische Sonde nach Anspruch 1 oder Anspruch 2, wobei der mindestens eine erste Lichtleiter (20) und der mindestens eine zweite Lichtleiter (22) jeweils einen länglichen Stab umfassen, der innerhalb eines jeweiligen Aufnahmekanals angebracht ist, der sich durch die Frontplatte (4) erstreckt.

4. Optische Sonde nach Anspruch 3, wobei jeder von dem mindestens einen ersten Lichtleiter (20) und dem mindestens einen zweiten Lichtleiter (22) dazu konfiguriert ist, die korrekte Positionierung und/oder Ausrichtung des jeweiligen Lichtleiters innerhalb seines Aufnahmekanals in der Frontplatte (4) zu ermöglichen.

5. Optische Sonde nach einem der vorhergehenden Ansprüche, wobei der Ultraschallwandler (8) über einen länglichen Körper (14) an die Frontplatte (4) gekoppelt ist, wobei mindestens ein distales Ende des länglichen Körpers (14) einen Durchmesser aufweist, der kleiner als der Durchmesser der Frontplatte (4) ist, sodass der längliche Körper (14) mit einem zentralen Bereich der Frontplatte (4) zusammenwirkt, um Ultraschallschwingungen auf diese zu übertragen.

6. Optische Sonde nach Anspruch 5, wobei sich der mindestens eine erste Lichtleiter (20) und der mindestens eine zweite Lichtleiter (22) in einem peripheren Bereich der Frontplatte (4) außerhalb des zentralen Bereichs derselben befinden.

7. Optische Sonde nach Anspruch 5 oder Anspruch 6, wobei der Ultraschallwandler (8) an einem ersten Ende des länglichen Körpers (14) angebracht ist, wobei ein zweites Ende des länglichen Körpers (14), das dem ersten Ende gegenüberliegt, an der Frontplatte (4) angebracht ist.

8. Optische Sonde nach Anspruch 7, wobei der Ultraschallwandler (8) an dem ersten Ende des länglichen Körpers (14) durch ein durch diesen verlaufendes Befestigungselement (16) gesichert ist.

9. Optische Sonde nach Anspruch 7 oder Anspruch 8, wobei das zweite Ende des länglichen Körpers (14) an der Frontplatte (4) durch ein durch diese verlaufendes Befestigungselement (18) gesichert ist.

10. Optische Sonde nach einem der Ansprüche 7 bis 9, wobei der Ultraschallwandler (8) einen Stapel von Wandlerscheiben (10) umfasst, die an eine Rückseitenmasse (12) gekoppelt sind.

11. Optische Sonde nach einem der vorhergehenden Ansprüche, wobei der mindestens eine erste Lichtleiter (20) und/oder der mindestens eine zweite Lichtleiter (22) angepasst sind, durch sie verlaufendes Licht zu modifizieren.

12. Optische Sonde nach Anspruch 11, wobei der mindestens eine erste Lichtleiter (20) und/oder der mindestens eine zweite Lichtleiter (22) mindestens eine Linse umfassen oder enthalten.

13. Optische Sonde nach Anspruch 12, wobei ein distales Ende jedes von dem mindestens einen ersten Lichtleiter (20) und dem mindestens einen zweiten Lichtleiter (22) angepasst ist, durch diese verlaufendes Licht zu modifizieren.

14. Optische Sonde nach Anspruch 13, wobei das distale Ende jedes von dem mindestens einen ersten Lichtleiter (20) und dem mindestens einen zweiten Lichtleiter (22) eine Fläche umfasst, die sich in einem spitzen Winkel zu der Achse des jeweiligen Lichtleiters und im Wesentlichen parallel zu der Frontfläche (6) der Frontplatte (4) erstreckt, und wobei der mindestens eine erste Lichtleiter (20) und der mindestens eine zweite Lichtleiter (22) innerhalb der Frontplatte (20) an jeweiligen Achsen angebracht sind, die so angeordnet sind, dass sie in einem gemeinsamen Punkt innerhalb des Messbereichs konvergieren.

15. Optische Sonde nach einem der vorhergehenden Ansprüche, wobei jeder von dem mindestens einen ersten Lichtleiter (20) und dem mindestens einen zweiten Lichtleiter (22) ein erstes Teil und ein zweites Teil umfasst, die koaxial angeordnet sind, wobei das erste Teil (20, 22) in der Frontplatte (4) angebracht ist, sodass ein distales Ende davon mit dem Messbereich in Verbindung steht, wobei das zweite Teil (24, 26) benachbart zu dem jeweiligen ersten Teil (20, 22) angebracht und koaxial mit diesem ausgerichtet ist, wobei ein Spalt (28, 30) zwischen benachbarten Enden des ersten und des zweiten Teils jedes von dem mindestens einen ersten Lichtleiter und dem mindestens einen zweiten Lichtleiter bereitgestellt ist, um dadurch das zweite Teil (24, 26) jedes von dem mindestens einen ersten Lichtleiter (20) und dem mindestens einen zweiten Lichtleiter (22) gegenüber Ultraschallenergie zu isolieren.

## Revendications

1. Sonde optique (2) comprenant une plaque frontale (4) présentant une face avant délimitant une zone de mesure, au moins un premier guide de lumière (20) étant monté à l'intérieur de ladite plaque frontale (4) pour transmettre la lumière directement dans ladite zone de mesure, au moins un second guide de lumière (22) étant monté à l'intérieur de ladite plaque frontale (4) pour recevoir la lumière transmise directement depuis ladite zone de mesure, dans laquelle un transducteur ultrasonore (8) est couplé à ladite plaque frontale (4) pour transmettre des vibrations ultrasonores dudit transducteur ultrasonore à ladite plaque frontale (4) le long d'un trajet énergétique afin de générer une cavitation dans ladite zone de mesure, dans laquelle lesdits au moins un premier guide de lumière (20) et au moins un second guide de lumière (22) se prolongent à travers la plaque frontale (4) à des emplacements respectifs espacés dudit trajet énergétique de sorte que lesdits premier et second guides de lumière (20, 22) ne sont pas exposés à l'énergie ultrasonore directe dudit transducteur ultrasonore (8) lorsqu'ils sont exposés à une zone de cavitation générée par le transducteur ultrasonore (8).

2. Sonde optique selon la revendication 1, dans laquelle lesdits au moins un premier guide de lumière (20) et au moins un second guide de lumière (22) sont montés à l'intérieur de ladite plaque frontale (4) sur des axes respectifs convergeant vers un point commun à l'intérieur de ladite zone de mesure.

3. Sonde optique selon la revendication 1 ou la revendication 2, dans laquelle ledit au moins un premier guide de lumière (20) et ledit au moins un second guide de lumière (22) comprennent chacun une tige allongée montée à l'intérieur d'un canal de réception respectif se prolongeant à travers ladite plaque frontale (4).

4. Sonde optique selon la revendication 3, dans laquelle chacun desdits au moins un premier guide de lumière (20) et au moins un second guide de lumière (22) est configuré pour faciliter l'emplacement et/ou l'orientation corrects du guide de lumière respectif à l'intérieur de son canal de réception dans la plaque frontale (4).

5. Sonde optique selon une quelconque revendication précédente, dans laquelle ledit transducteur ultrasonore (8) est couplé à ladite plaque frontale (4) par l'intermédiaire d'un corps allongé (14), au moins une extrémité distale dudit corps allongé (14) présentant un diamètre inférieur au diamètre de ladite plaque frontale (4) de sorte que ledit corps allongé (14) coopère avec une zone centrale de ladite plaque avant (4) pour lui transmettre des vibrations ultrasonores.

6. Sonde optique selon la revendication 5, dans laquelle ledit au moins un premier guide de lumière (20) et ledit au moins un second guide de lumière (22) sont situés à l'intérieur d'une zone périphérique de ladite plaque frontale (4) à l'extérieur de ladite zone centrale de celle-ci.

7. Sonde optique selon la revendication 5 ou la revendication 6, dans laquelle ledit transducteur ultrasonore (8) est monté contre une première extrémité dudit corps allongé (14), une seconde extrémité dudit corps allongé (14), opposée à ladite première extrémité, étant montée contre ladite plaque frontale (4).

8. Sonde optique selon la revendication 7, dans laquelle ledit transducteur ultrasonore (8) est fixé à ladite première extrémité dudit corps allongé (14) au moyen d'une fixation (16) le traversant.

9. Sonde optique selon la revendication 7 ou la revendication 8, dans laquelle ladite seconde extrémité dudit corps allongé (14) est fixée à ladite plaque frontale (4) au moyen d'une fixation (18) la traversant.

10. Sonde optique selon l'une quelconque des revendications 7 à 9, dans laquelle ledit transducteur ultrasonore (8) comprend un empilement de disques transducteurs (10) couplés à une masse arrière (12).

11. Sonde optique selon une quelconque revendication précédente, dans laquelle ledit au moins un premier guide de lumière (20) et/ou ledit au moins un second guide de lumière (22) est adapté pour modifier la lumière qui le traverse.

12. Sonde optique selon la revendication 11, dans laquelle ledit au moins un premier guide de lumière (20) et/ou ledit au moins un second guide de lumière (22) comprend ou incorpore au moins une lentille.

13. Sonde optique selon la revendication 12, dans laquelle une extrémité distale de chacun dudit au moins un premier guide de lumière (20) et dudit au moins un second guide de lumière (22) est adaptée pour modifier la lumière qui la traverse.

14. Sonde optique selon la revendication 13, dans laquelle ladite extrémité distale de chacun dudit au moins un premier guide de lumière (20) et dudit au moins un second guide de lumière (22) comprend une face se prolongeant à un angle aigu par rapport à l'axe du guide de lumière respectif et sensiblement parallèle à ladite face frontale (6) de la plaque frontale (4), et dans laquelle ledit au moins un premier guide de lumière (20) et ledit au moins un second guide de lumière (22) sont montés à l'intérieur de ladite plaque frontale (20) sur des axes respectifs agencés de manière à converger sur un point commun à l'intérieur de la zone de mesure.

15. Sonde optique selon l'une quelconque des revendications précédentes, dans laquelle chacun dudit au moins un premier guide de lumière (20) et dudit au moins un second guide de lumière (22) comprend une première et une seconde parties agencées coaxialement, la première partie (20, 22) étant montée dans la plaque frontale (4) de sorte qu'une extrémité distale de celle-ci communique avec la zone de mesure, la seconde partie (24, 26) étant montée à côté et alignée coaxialement avec la première partie respective (20, 22), un espace (28, 30) étant fourni entre des extrémités adjacentes des première et seconde parties de chacun dudit au moins un premier guide de lumière et dudit au moins un second guide de lumière afin d'isoler la seconde partie (24, 26) de chacun dudit au moins un premier guide de lumière (20) et dudit au moins un second guide de lumière (22) de l'énergie ultrasonore.
